# EUROPEAN PATENT APPLICATION

(11) **EP 3 673 812 A1**
(43) Date of publication of application: **01.07.2020**
(21) Application number: 19155996.2
(22) Date of filing: 07.02.2019
(51) Int. Cl.: A61B 8/08, A61B 17/34

(54) **BRACKET AND NEEDLE GUIDE FOR ULTRASONIC PROBE**

(30) Priority: 28.12.2018 KR 20180172446
(71) Applicant: RF Medical Co., Ltd., Seoul 08511 (KR)
(72) Inventor: JUN, Myong Ki, Gyeonggi-do 14244 (KR)
(74) Representative: Müller Hoffmann & Partner

(57) **Abstract**

The present invention relates to a bracket and a needle guide for an ultrasonic probe. The bracket and the needle guide for the ultrasonic probe according to the present invention include: a bracket formed to enclose the ultrasonic probe; at least one projection formed on the inside of the bracket; a needle guide body formed integrally with the bracket on one side of the bracket; a slot formed in the needle guide body so that a needle can be inserted thereinto and rotated therein; a cutout groove vertically formed on the lateral surface of the needle guide body to communicate with the slot; and an outlet through which the needle passing through the slot is drawn out of the needle guide body.

## Description

### TECHNICAL FIELD

The present invention relates to a bracket and a needle guide for an ultrasonic probe. According to the present invention, a two-dimensional degree of freedom is provided to a needle guide having a fixed guide direction, which makes it possible to guide a needle at various angles and positions in an ultrasonic plane, and a cutout groove or an open face for guiding the needle is formed in the needle guide, which makes it possible to prevent the needle from being damaged during the operation.

### BACKGROUND ART

In high frequency thermal therapy or biopsy for thyroid tumors, in terms of treatment techniques, an operator puts an ultrasonic probe on the skin near the tumor and inserts a high frequency electrode while looking at an ultrasonic image. A general ultrasonic probe provides a two-dimensional image. An ultrasonic probe showing a three-dimensional image is useful for diagnostic purposes but not suitable for therapeutic purposes due to its large volume, so it is not generally employed.

Meanwhile, if the high frequency electrode or biopsy needle gets out of the ultrasonic image plane, it may damage a normal tissue or organ and may lead to death in severe cases. It is thus necessary to target the electrode or needle not to get out of the ultrasonic image plane, which is very difficult for a less-experienced operator. In order to avoid such risks, operators practice with models but do not have a sufficient time for the practice, such that patients are exposed to danger of medical accidents. Even operators perform the operation with uneasiness. For this reason, generally in biopsy, a needle guide which is a needle guiding device is attached to an ultrasonic probe and a needle advance angle is set in a program of an ultrasonic equipment.

However, the conventional needle guide is designed such that a needle is always inserted at a single angle or at one of several angles. Therefore, in order to adopt a different angle, it is always required to adjust a lever. In high frequency thermal therapy, the operator rapidly moves the electrode to partially perform the cautery on the tumor, which eventually cauterizes the whole tumor. To cauterize the tumor at a time by piercing the center of the tumor with an electrode having a high capability for the cautery is impractical because it is risky and not suitable for irregular-shaped tumors. Until now, instead of using such a needle guide, the operator takes hold of the ultrasonic probe with one hand and the electrode with the other hand and keeps moving the electrode to perform the cautery, while preventing the electrode from getting out of the ultrasonic image plane with a three-dimensional sense. This cautery method is referred to as a moving shot technique. It takes operators some time to get into their stride using the moving shot technique, so patients are still exposed to danger. From a medial point of view, to mistakenly target the needle and cauterize another part is the most common complication of the high frequency thermal therapy for thyroid tumors.

In addition, the operator inserts the needle into the needle guide to start the operation. Typically, since the needle has a very small diameter and includes a micro-machined tip, even a slight impact may break the needle or wear the tip. The conventional needle guide is not provided with any configuration for protecting or guiding the needle having the micro structure, such that the needle may be damaged during the operation or the operator may inefficiently perform the cautery without noticing the damaged needle.

Conventionally, Korea Laid-Open Patent Publication Nos. 10-2001-0032747 and 10-2015-0133449 disclose configurations related to an ultrasonic probe and a needle guide, which do not include a configuration for preventing a needle from being damaged when inserted into the needle guide and for protecting the needle.

In order to solve the foregoing problems, the present invention relates to a device designed to show an electrode in a two-dimensional plane all the time and to allow an insertion angle to be freely changed. The device according to the present invention includes a bracket manufactured suitable for an ultrasonic probe and a needle guide formed integrally with the bracket, such that, when they are mounted on the ultrasonic probe, the electrode always exists in the ultrasonic image, as a result of which it will never happen that an operator cauterizes a normal tissue or organ without noticing that the needle is in a wrong position. According to the present invention, it is easy and safe to collect tissues from a few parts of one tumor. Further, it is also possible to prevent the needle from being damaged when inserted into the needle guide, which maintains the effectiveness of the cautery.

### DISCLOSURE OF THE INVENTION

The present invention has been made to solve the foregoing problems. One object of the present invention is to provide a two-dimensional degree of freedom to a needle guide to guide a needle at various angles and positions in an ultrasonic plane.

Another object of the present invention is to provide a bracket manufactured suitable for an ultrasonic probe and a needle guide formed integrally with the bracket to stably perform an operation.

A further object of the present invention is to provide a configuration for preventing a needle from being damaged when inserting the needle into a needle guide.

A still further object of the present invention is to provide a needle guide configuration for significantly reducing the risk of damaging an insulating film coated on a needle.

In order to achieve the above objects, the present invention provides the following embodiments.

According to an aspect of the present invention, there are provided a bracket and a needle guide for an ultrasonic probe, including: a bracket formed to enclose the ultrasonic probe; at least one projection formed on the inside of the bracket; a needle guide body formed integrally with the bracket on one side of the bracket; a slot formed in the needle guide body so that a needle can be inserted thereinto and rotated therein; a cutout groove vertically formed on the lateral surface of the needle guide body to communicate with the slot; and an outlet through which the needle passing through the slot is drawn out of the needle guide body.

In some embodiments, at least one needle guiding projection is formed on the lateral surface of the needle guide body.

In some embodiments, the outlet is formed in a slit shape.

In some embodiments, the rotational angle of the needle rotated through the slot of the needle guide ranges from 0 to 70 degrees.

In some embodiments, at least one stopping projection is formed at one side of the bracket and a receiving groove with which the stopping projection is fixedly engaged is formed in the needle guide body.

According to another aspect of the present invention, there is provided a needle guide for an ultrasonic probe, including: a needle guide body; a slot formed in the needle guide body so that a needle can be inserted thereinto and rotated therein; an open face formed on the needle guide body so that the needle can be rotated thereon; a needle guiding face formed on the needle guide body; and an outlet through which the needle is drawn out of the needle guide body.

According to a further aspect of the present invention, there is provided a needle guide for an ultrasonic probe, including: a needle guide body; a sidewall formed on the needle guide body; an open face formed on the needle guide body so that the needle can be rotated thereon; a needle guiding face formed on the needle guide body; and an outlet through which the needle is drawn out of the needle guide body.

As described above, according to the present invention, the needle can freely move in two dimensions around the outlet on the inside of the needle guide, which makes it possible for the needle guide to control the needle with a much higher degree of freedom than the conventional needle guide having a fixed guide direction.

In addition, while the conventional bracket and needle guide are separate, the bracket and the needle guide according to the present invention are integrally formed suitable for the corresponding probe, which makes it possible to firmly fix the probe using the bracket and reduce the number of the parts.

Moreover, according to the present invention, the cutout groove for guiding the needle is formed in the needle guide, which makes it possible to prevent the needle from being damaged.

Further, according to the present invention, the open face is formed on the needle guide, which makes it possible to prevent the needle from being damaged in the operation.

Furthermore, according to the present invention, the open face is formed on the needle guide, which makes it possible to easily change the direction of the needle in the operation, which leads to flexible handling based on situations.

Still furthermore, according to the present invention, an operator can insert the needle into a cautery region at a certain angle, thereby significantly reducing the possibility of damaging the needle, and also can perform the cautery on the cautery regions divided at certain intervals, thereby significantly reducing the possibility of skipping the affected part.

### BRIEF DESCRIPTION OF THE DRAWINGS

FIG. 1 is a perspective view showing one embodiment of a bracket and a needle guide for an ultrasonic probe according to the present invention, when seen from the top.
FIG. 2 is a perspective view showing one embodiment of the bracket and the needle guide for the ultrasonic probe according to the present invention, when seen from the bottom.
FIG. 3 is a side view showing the needle guide in one embodiment of the bracket and the needle guide for the ultrasonic probe according to the present invention.
FIG. 4 is a top view showing the needle guide in one embodiment of the bracket and the needle guide for the ultrasonic probe according to the present invention.
FIG. 5 is a perspective view showing another embodiment of a bracket and a needle guide for an ultrasonic probe according to the present invention.
FIG. 6 is a side view showing the needle guide in another embodiment of the bracket and the needle guide for the ultrasonic probe according to the present invention.
FIG. 7 is a perspective view showing a further embodiment of major parts of a bracket and a needle guide for an ultrasonic probe according to the present invention.
FIG. 8 is a side view showing the further embodiment of the major parts of the bracket and the needle guide for the ultrasonic probe according to the present invention.
FIG. 9 is a perspective view showing a needle guide coupled to a bracket as one example of the needle guide for the ultrasonic probe according to the present invention.
FIG. 10 is a perspective view showing a needle guide coupled to a bracket as another example of the needle guide for the ultrasonic probe according to the present invention.
FIG. 11 is a perspective view showing a needle guide coupled to a bracket as a further example of the needle guide for the ultrasonic probe according to the present invention.

### BEST MODE FOR CARRYING OUT THE INVENTION

Hereinafter, preferred embodiments of a bracket and a needle guide for an ultrasonic probe according to the present invention will be described in detail with reference to the accompanying drawings.

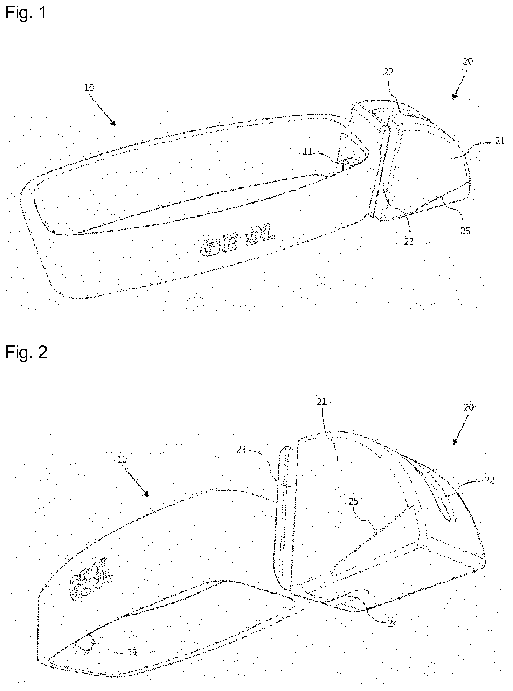

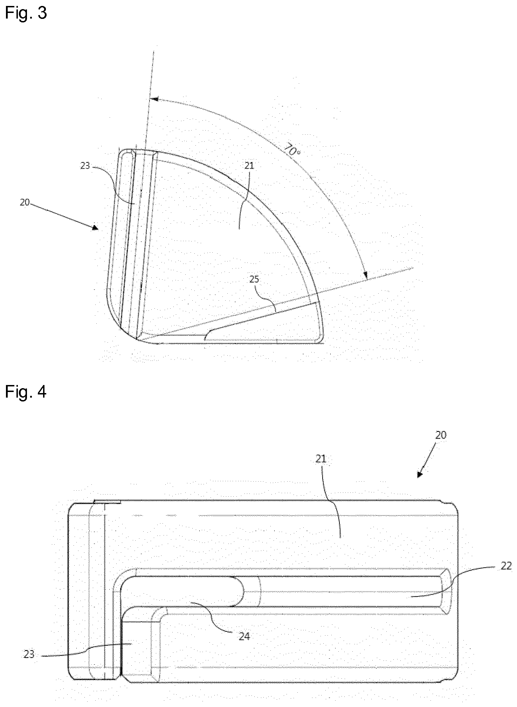

FIG. 1 is a perspective view showing one embodiment of a bracket and a needle guide for an ultrasonic probe according to the present invention, when seen from the top. FIG. 2 is a perspective view showing one embodiment of the bracket and the needle guide for the ultrasonic probe according to the present invention, when seen from the bottom. FIG. 3 is a side view showing the needle guide in one embodiment of the bracket and the needle guide for the ultrasonic probe according to the present invention. FIG. 4 is a top view showing the needle guide in one embodiment of the bracket and the needle guide for the ultrasonic probe according to the present invention. Referring to FIGS. 1 to 4, the bracket and the needle guide for the ultrasonic probe according to one embodiment of the present invention include: a bracket 10 formed to enclose the ultrasonic probe; at least one projection 11 formed on the inside of the bracket 10; a needle guide body 21 formed integrally with the bracket 10 on one side of the bracket 10; a slot 22 formed in the needle guide body 21 so that a needle can be inserted thereinto and rotated therein; a cutout groove 23 vertically formed on the lateral surface of the needle guide body 21 to communicate with the slot 22; and an outlet 24 through which the needle passing through the slot 22 is drawn out of the needle guide body 21. The bracket 10 formed to enclose the ultrasonic probe may be manufactured to fit onto an ultrasonic probe to be used, may be made of a plastic, and in some embodiments, may be made of a variety of different materials such as polyethylene, polypropylene, etc. At least one projection 11 is formed on an inner portion with a short diameter on the inside of the bracket 10, and it is also possible for two or more projections 11 to be formed in opposite positions on the inside of the bracket 10. In general, grooves corresponding to the projections 11 are formed in the ultrasonic probe. Therefore, when the bracket 10 is fit onto the ultrasonic probe, the projections 11 are fit into the grooves formed in the ultrasonic probe, such that the ultrasonic probe is firmly fastened and fixed to the bracket 10. It is thus possible to prevent the ultrasonic probe from moving or separating from the bracket 10 during the operation using the ultrasonic probe. The needle guide body 21 is integrally formed on one side of the bracket 10, and in some embodiments, the needle guide body 21 may be manufactured integrally with the bracket 10. The slot 22 is formed in the top surface of the needle guide body 21 so that the needle can be inserted thereinto and rotated therein, the length and width of the slot 22 being adjustable if desired. In addition, the cutout groove 23 is vertically formed on the lateral surface of the needle guide body 21 to communicate with the slot 22. Accordingly, when an operator intends to insert a needle for the purpose of cautery, the operator does not insert the needle into the slot 22 directly in the vertical direction of the needle guide 20, but inserts the needle into the slot 22 through the cutout groove 23 on the lateral surface of the needle guide body 21. As the cutout groove 23 serves to guide the needle into the slot 22, the needle can be stably inserted into the slot 22, thereby significantly reducing the possibility of damaging or breaking the needle or wearing its tip. The needle inserted into the slot is drawn out through the outlet 24 formed in a lower portion of the needle guide body 21 and used to cauterize the affected part.

According to one embodiment of the present invention, the bracket and the needle guide for the ultrasonic probe are characterized in that at least one needle guiding projection 25 is formed on the lateral surface of the needle guide body 21. The needle guiding projection 25 serves to guide the range of movement of the needle inserted into the slot 22, while the operator performs the cautery. As a result, the operator can properly adjust the range of movement of the needle in the needle guide 20, whenever moving to another part to be cauterized, and stably move the needle to perform the cautery.

According to one embodiment of the present invention, the bracket and the needle guide for the ultrasonic probe are characterized in that the outlet 24 is formed in a slit shape. The slit-shaped outlet 24 makes it possible to adjust the angle of the needle projecting through the outlet 24 a little more, which leads to the improved degree of freedom in cautery. The length and width of the slit is adjustable, if desired.

According to one embodiment of the present invention, the bracket and the needle guide for the ultrasonic probe are characterized in that the rotational angle of the needle rotated through the slot 22 of the needle guide 20 ranges from 0 to 70 degrees. The rotational angle of the needle can be controlled by adjusting the length of the slot 22. Empirically, it is typically preferred that the rotational angle of the needle ranges from 0 to 70 degrees in terms of ease of stable handling of the needle in the operation.

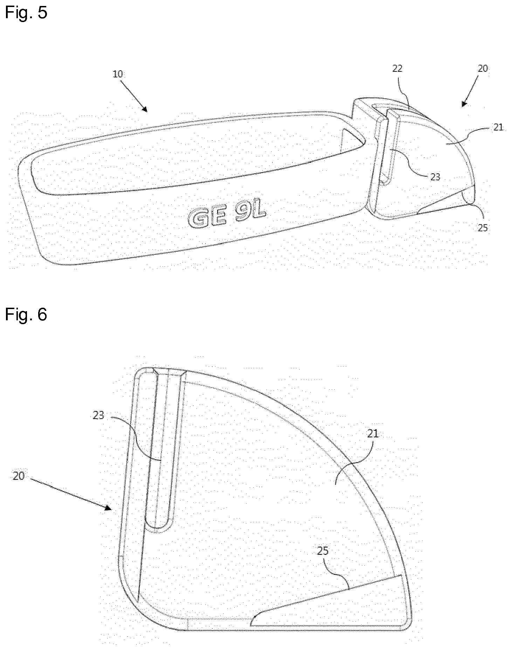

FIG. 5 is a perspective view showing another embodiment of a bracket and a needle guide for an ultrasonic probe according to the present invention. FIG. 6 is a side view showing the needle guide in another embodiment of the bracket and the needle guide for the ultrasonic probe according to the present invention. In the embodiment described with reference to FIGS. 1 to 4, the cutout groove 23 formed in the needle guide 20 extends from the upper end to the lower end of the needle guide body 21 in the vertical direction, communicating with the slot 22 formed in the upper portion of the needle guide 20. The cutout groove 23 formed in that manner may affect the rigidity of the needle guide body 21. Referring to FIGS. 5 and 6, the cutout groove 23 is still formed in the needle guide body 21 in the vertical direction, but is formed partially open with respect to the vertical length of the needle guide body 21. Thus, the cutout groove 23 formed in this manner can serve to guide the needle without affecting the rigidity of the needle guide body 21. In some embodiments, preferably, the cutout groove 23 occupies less than 2/3 of the vertical length of the needle guide body 21.

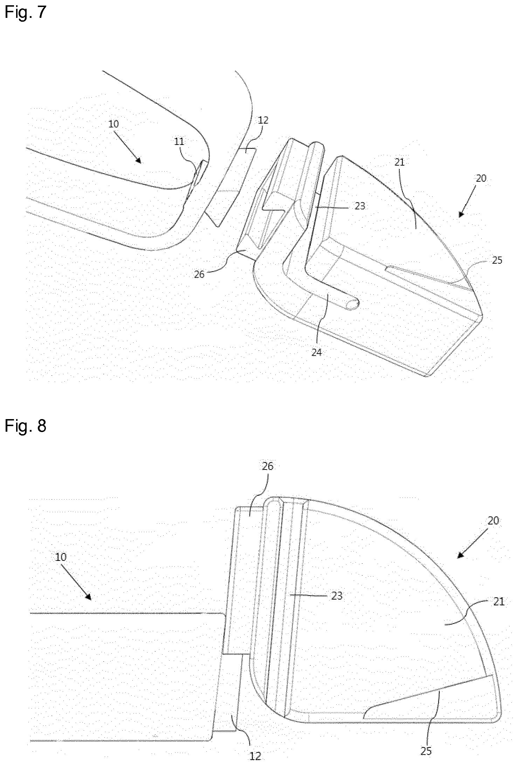

FIG. 7 is a perspective view showing a further embodiment of major parts of a bracket and a needle guide for an ultrasonic probe according to the present invention. FIG. 8 is a side view showing the further embodiment of the major parts of the bracket and the needle guide for the ultrasonic probe according to the present invention. Referring to FIGS. 7 and 8, according to one embodiment of the present invention, the bracket and the needle guide for the ultrasonic probe are characterized in that at least one stopping projection 12 is formed at one side of the bracket 10 and a receiving groove 26 with which the stopping projection 12 is fixedly engaged is formed in the needle guide body 21. In the above-described embodiment of the present invention, the bracket and the needle guide body for the ultrasonic probe have been formed as a single body. However, if need be, the stopping projection 12 is formed at one side of the bracket 10, and the receiving groove 26 with which the stopping projection 12 is fixedly engaged is formed in the needle guide body 21, such that the bracket and the needle guide body for the ultrasonic probe can be fastened to each other in use and separated from each other after use. As a result, it is possible to manufacture only the bracket 10 and couple it to the needle guide 20 manufactured in advance, instead of manufacturing both the bracket 10 and the needle guide 20 depending on the size of the ultrasonic probe. The receiving groove 26 may be vertically formed in the needle guide body 21 in a projecting manner, and the stopping projection 12 may be formed at one side of the bracket 10 in a manner corresponding thereto.

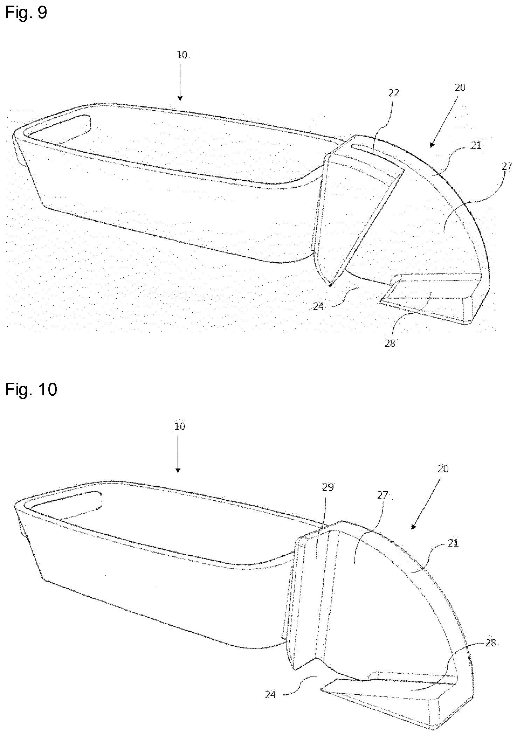

FIG. 9 is a perspective view showing a needle guide coupled to a bracket as one example of the needle guide for the ultrasonic probe according to the present invention. Referring to FIG. 9, the needle guide for the ultrasonic probe according to one embodiment of the present invention includes a needle guide body 21, a slot 22 formed in the needle guide body 21 so that a needle can be inserted thereinto and rotated therein, an open face 27 formed on the needle guide body 21 so that the needle can be rotated thereon, a needle guiding face 28 formed on the needle guide body 21, and an outlet 24 through which the needle is drawn out of the needle guide body 21. The needle guide 20 is coupled to and used with the bracket 10 formed to enclose the ultrasonic probe in the operation. In some embodiments, the needle guide body 21 may be integrally formed at one side of the bracket 10. In some embodiments, the open face 27 formed on the needle guide body 21 is formed between the slot 22 and the needle guiding face 28 of the needle guide body 21 and the open face 27 communicates with the slot 22. Therefore, when an operator uses the needle for the purpose of cautery, the operator does not insert the needle in the vertical direction of the needle guide 20, but puts the needle onto the open face 27, such that the needle can be guided by the open face 27 and rotated along the open face 27 and the slot 22. The needle put onto the open face 27 and rotated along the open face 27 and the slot 22 is drawn out through the outlet 24 formed in a lower portion of the needle guide body 21 and used to cauterize the affected part. As a result, the operator can properly adjust the range of movement of the needle in the needle guide 20, whenever moving to another part to be cauterized, and stably move the needle to perform the cautery, which makes it possible to significantly reduce the possibility of damaging or breaking the needle or wearing its tip in the operation. Meanwhile, even if the needle is inserted in the vertical direction of the needle guide 20, the open face 27 allows the needle to be inserted at various angles. When the operator changes the direction of the needle in the operation, the operator can readily handle the needle even in the vertical direction of the needle guide 20. In some embodiments, the open face 27 formed on the needle guide body 21 may be formed between the slot 22 and the needle guiding face 28 of the needle guide body 21 within a range of 30 to 70 degrees relative to the slot 22 and the angle of the open face 27 may be arbitrarily chosen within this range in terms of ease of stable handling of the needle in the operation.

FIG. 10 is a perspective view showing a needle guide coupled to a bracket as another example of the needle guide for the ultrasonic probe according to the present invention. Referring to FIG. 10, the needle guide for the ultrasonic probe according to the present invention includes a needle guide body 21, a sidewall 29 formed on the needle guide body 21, an open face 27 formed on the needle guide body 21 so that the needle can be rotated thereon, a needle guiding face 28 formed on the needle guide body 21, and an outlet 24 through which the needle is drawn out of the needle guide body 21. In some embodiments, the open face 27 is formed between the sidewall 29 and the needle guiding face 28 of the needle guide body 21. Therefore, when an operator uses the needle for the purpose of cautery, the operator does not insert the needle in the vertical direction of the needle guide 20, but puts the needle onto the open face 27, such that the needle can be guided and rotated by the open face 27. The needle guiding face 28 serves to guide the range of rotation of the needle put onto the open face 27, and the needle put onto the open face 27 and rotated along the open face 27 is drawn out through the outlet 24 formed in a lower portion of the needle guide body 21 and used to cauterize the affected part. As a result, the operator can properly adjust the range of movement of the needle in the needle guide 20, whenever moving to another part to be cauterized, and stably move the needle to perform the cautery, which makes it possible to significantly reduce the possibility of damaging or breaking the needle or wearing its tip in the operation. Meanwhile, even if the needle is inserted in the vertical direction of the needle guide 20, the open face 27 allows the needle to be inserted at various angles. When the operator changes the direction of the needle in the operation, the operator can readily handle the needle even in the vertical direction of the needle guide 20. In some embodiments, the open face 27 formed on the needle guide body 21 so that the needle can be rotated thereon may be formed between the sidewall 29 and the needle guiding face 28 of the needle guide body 21 at an angle of 70 degrees or more relative to the sidewall 29 in terms of the range of rotation of the needle and ease of handling of the needle.

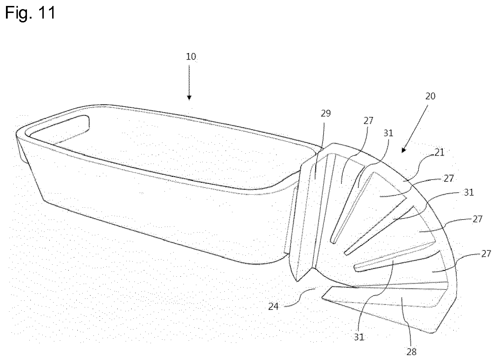

FIG. 11 is a perspective view showing a needle guide coupled to a bracket as a further example of the needle guide for the ultrasonic probe according to the present invention. Referring to FIG. 11, in the needle guide for the ultrasonic probe according to the present invention, a plurality of guiding projections 31 are formed on an open face 27 formed on a needle guide body 21 so that the needle can be rotated thereon. For example, eight guiding projections 31 may be formed between a sidewall 29 and a needle guiding face 28 of the needle guide body 21 at intervals of 10 degrees to divide cautery regions. Since the needle can be inserted into the intervals divided by the guiding projections 31, the needle can be positioned and guided on the ultrasonic plane which is the cautery region to perform the cautery at certain intervals. Accordingly, even a less-experienced operator can insert the needle into the cautery region at a certain angle, thereby significantly reducing the possibility of damaging the needle, without the aid of a separate device, and also can perform the cautery on the cautery regions divided at certain intervals, thereby significantly reducing the possibility of skipping the affected part. The intervals of the guiding projections 31 between the sidewall 29 and the needle guiding face 28 of the needle guide body 21 depend on the size of the cautery region. For example, the guiding projections 31 may be formed at intervals of 20 degrees for a small tumor, 15 degrees for a middle tumor, and 10 degrees for a big tumor. If need be, the intervals of the guiding projections may vary.

While the present invention has been illustrated and described in connection with the accompanying drawings and the preferred embodiments, the present invention is not limited thereto and is defined by the appended claims. Therefore, it will be understood by those skilled in the art that various modifications and changes can be made thereto without departing from the spirit and scope of the invention defined by the appended claims.

### [Description on Reference Numerals]

- 10 :: bracket
- 11 :: projection
- 12 :: stopping projection
- 20 :: needle guide
- 21 :: needle guide body
- 22 :: slot
- 23 :: cutout groove
- 24 :: outlet
- 25 :: needle guiding projection
- 26 :: receiving groove
- 27 :: open face
- 28 :: needle guiding face
- 29 :: sidewall
- 31 :: guiding projection

## Claims

1. A bracket and a needle guide for an ultrasonic probe, comprising:
a bracket formed to enclose the ultrasonic probe;
at least one projection formed on the inside of the bracket;
a needle guide body formed integrally with the bracket on one side of the bracket;
a slot formed in the needle guide body so that a needle can be inserted thereinto and rotated therein;
a cutout groove vertically formed on the lateral surface of the needle guide body to communicate with the slot; and
an outlet through which the needle passing through the slot is drawn out of the needle guide body.

2. The bracket and the needle guide of claim 1, wherein at least one needle guiding projection is formed on the lateral surface of the needle guide body.

3. The bracket and the needle guide of claim 1, wherein the outlet is formed in a slit shape.

4. The bracket and the needle guide of claim 1, wherein the rotational angle of the needle rotated through the slot of the needle guide ranges from 0 to 70 degrees.

5. The bracket and the needle guide of claim 1, wherein the bracket is made of polyethylene or polypropylene.

6. The bracket and the needle guide of claim 1, wherein the cutout groove is formed partially open with respect to the vertical length of the needle guide body.

7. The bracket and the needle guide of claim 1, wherein at least one stopping projection is formed at one side of the bracket and a receiving groove with which the stopping projection is fixedly engaged is formed in the needle guide body.

8. A needle guide for an ultrasonic probe, comprising:
a needle guide body;
a slot formed in the needle guide body so that a needle can be inserted thereinto and rotated therein;
an open face formed on the needle guide body so that the needle can be rotated thereon;
a needle guiding face formed on the needle guide body; and
an outlet through which the needle is drawn out of the needle guide body.

9. The needle guide of claim 8, wherein the open face formed on the needle guide body so that the needle can be rotated thereon is formed between the slot and the needle guiding face.

10. The needle guide of either claim 8 or 9, wherein the open face formed on the needle guide body so that the needle can be rotated thereon is formed between the slot and the needle guiding face within a range of 30 to 70 degrees relative to the slot.

11. A needle guide for an ultrasonic probe, comprising:
a needle guide body;
a sidewall formed on the needle guide body;
an open face formed on the needle guide body so that the needle can be rotated thereon;
a needle guiding face formed on the needle guide body; and
an outlet through which the needle is drawn out of the needle guide body.

12. The needle guide of claim 11, wherein the open face formed on the needle guide body so that the needle can be rotated thereon is formed between the sidewall and the needle guiding face.

13. The needle guide of either claim 11 or 12, wherein the open face formed on the needle guide body so that the needle can be rotated thereon is formed between the sidewall and the needle guiding face at an angle of 70 degrees or more relative to the sidewall.

14. The needle guide of either claim 11 or 12, wherein a plurality of guiding projections are formed on the open face formed on the needle guide body so that the needle can be rotated thereon.

15. The needle guide of claim 14, wherein the plurality of guiding projections are formed between the sidewall and the needle guiding face at intervals of 10 to 20 degrees.
